# EUROPEAN PATENT APPLICATION

(11) **EP 1 820 443 A1**
(43) Date of publication of application: **22.08.2007**
(21) Application number: 07250612.4
(22) Date of filing: 15.02.2007
(51) Int. Cl.: A61B 5/024

(54) **Pulse measuring apparatus**

(30) Priority: 16.02.2006 JP 2006039477
(71) Applicant: SEIKO INSTRUMENTS INC., Chiba-shi, Chiba (JP)
(72) Inventor: Nakamura, Hisao, Mihama-ku, Chiba-shi Chiba (JP)
(74) Representative: Cloughley, Peter Andrew

(57) **Abstract**

A pulse measuring apparatus possessing a pulse sensor unit (2), a timepiece head (1) and an arm wearing band (3). The pulse sensor unit (2) radio-transmits a pulse detection signal having been detected by a pulse sensor (25). A timepiece head (1) receives the pulse detection signal, and displays processed a pulse number. The pulse sensor unit is attached to a band piece for arm wearing so as to be movable in a longitudinal direction. Electricity is supplied to the pulse sensor unit (2) from a battery accommodated in the timepiece head (1) by wiring (4) embedded in the arm wearing band (3). The connection between the pulse sensor unit (2) and the wiring is established at exposed parts of the wiring (4), which have been formed at the bottom faces of concave parts for position adjustment (49). These are formed at an equal interval in the longitudinal direction in both sides of a slit (48) present in the arm wearing band (3). Said exposed parts correspond to and a position variable connection part having been constituted by one pair of electrically conductive connection pins (46) and one pair of electrically conductive pressurized springs (45), being seated in the sensor housing.

## Description

The present invention relates to a pulse measuring apparatus, a so-called wristwatch type pulse measuring apparatus, which comprises a pulse sensor having been accommodated in a sensor housing, a pulse measuring instrument in which a display section processing a detection signal of the pulse sensor to thereby display a pulse number has been accommodated in a measuring instrument case, and an arm wearing band which has been fixed to a measuring instrument case and to which the unit case has been attached so as to be adjustable in a longitudinal direction.

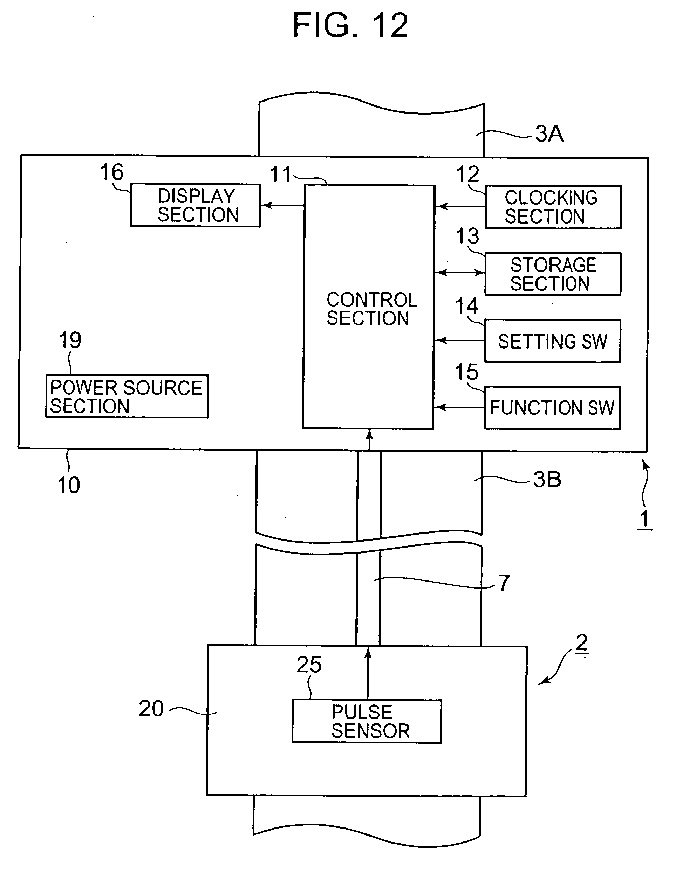

In order to always measure the pulse number during a motion and the like to there by make it an aim of a momentum, the wristwatch type pulse measuring apparatus has been developed. As shown in a block constitution diagram of Fig. 12, a conventional wristwatch type pulse measuring apparatus is constituted by a pulse measuring instrument 1, a pulse sensor unit 2, an arm wearing band 3A, 3B, and a signal line 7. The pulse measuring instrument 1 is constituted by a control section 11, a clocking section 12, a storage section 13, a display section 16, a setting switch 14, a function switch 15, a power source section 19, and a measuring instrument case 10 accommodating these constitutional elements.

The pulse sensor unit 2 is constituted by a pulse sensor 25, and a sensor housing 20 accommodating the pulse sensor 25.

The arm wearing band 3 comprises one pair of band pieces 3A and 3B. One end of one band piece 3A is fixed to one band attaching part of the measuring instrument case 10, and one end of the other band piece 3B to the other band attaching part of the measuring instrument case 10, respectively. And, the pulse sensor unit 2 is attached to the band piece 3B so as to be adjustable in the longitudinal direction. A position adjusting function having been made so as to be capable of adjusting an attaching position of the pulse sensor unit 2 on the band is one provided while corresponding to the fact that a thickness of wrist and a position of artery of a user respectively differ, and it is a function indispensable for the wristwatch type pulse measuring apparatus.

Further, as mentioned above, in the wristwatch type pulse measuring apparatus, since the pulse sensor unit 2 is attached onto the band piece 3B while separating by about 10 cm from the pulse measuring instrument 1, in order to transmit a detection signal of the pulse sensor 25 of the pulse sensor unit 2 to the pulse measuring instrument 1 and input it to the control section 11, a signal transmission means is necessary. In Fig. 12, the signal transmission means is shown by the signal line 7 connecting the pulse sensor unit 2 and the pulse measuring instrument 1. Hereunder, there is explained about a concrete structure of the conventional wristwatch type pulse measuring apparatus.

As disclosed in JP-A-2002-143107 Gazette, a 1st conventional wristwatch type pulse measuring apparatus is one which has a band attached to a wristwatch case and extending from both ends of the case, an attaching long hole formed in the band and extending in a band longitudinal direction, and apulsesensor, and which comprises a sensor housing compressible and having been slidably attached to the attaching long hole, and a signal conduction means electrically connecting the pulse sensor to a signal processing circuit in the wristwatch case.

Although this 1st conventional wristwatch type pulse measuring apparatus is one bringing about an advantage that the pulse sensor that is a pulse detecting section is adjusted for an individual user to his/her optimum position, since it is a structure in which the pulse sensor is connected to a processor section in the wristwatch case through the signal conduction means, such as metal line, having been exposed in a band side face, there is a fear that the pulse measuring apparatus malfunctions while undergoing an influence of static electricity. That is, this is because, in a case where the sensor housing has been moved along the longitudinal direction of the band, the static electricity generated by a friction between a sensor unit or the band and a clothing of the user is transmitted to a semiconductor integrated circuit constituting the pulse sensor and the processor section through the signal conduction means, such as metal line, having been exposed in the band side face, so that there is the fact that these are destroyed.

As disclosed in JP-A-2005-40259 Gazette, a 2nd conventional wristwatch type pulse measuring apparatus is one characterized in that --in a wristwatch with a pulse number measuring device having possessed a pulse sensor, a band fixing this pulse sensor to a wrist, and a processor section having been accommodated in a timepiece case-- the pulse sensor has been attached to an inner side of the band so as to be slidable along a longitudinal direction of the band, and the pulse sensor and the processor section have been connected by a signal cable.

This 2nd conventional wristwatch type pulse measuring apparatus is also one bringing about the advantage that the pulse sensor that is the pulse detecting section is adjusted for an individual user to his/her optimum position. Further, since the signal cable connecting the pulse sensor and a signal processing circuit in the timepiece case are not exposed, there is also no problem of the destruction of the semiconductor integrated circuit and the like by the static electricity, which occurs in the above 1st conventional wristwatch type pulse measuring apparatus. However, if the pulse sensor is moved to a position having approached a timepiece case side, the signal cable generates slack. Thereupon, there occurs the fact that this signal cable having slack is caught by something, so that there has existed such a problem that the signal cable is impaired or a socket of the signal cable is detached from the timepiece case. In short, there has existed a problem that such a situation occurs that the signal transmission means undergoes a physical force, so that the signal from the pulse sensor becomes not transmitted to the processor section.

A problem that the present invention ought to solve is to make such that, in a pulse measuring apparatus comprising a pulse sensor unit in which a pulse sensor has been accommodated in a sensor housing, a pulse measuring instrument possessing a display section displaying a pulse number, and an arm wearing band which has been fixed to the pulse measuring instrument and to which the pulse sensor unit has been attached so as to be adjustable in a longitudinal direction, a signal is certainly transmitted to the pulse measuring instrument without a signal transmission means from the pulse sensor unit to the pulse measuring instrument being subjected to a physical adverse effect, and without a detection signal of the pulse sensor unit being subjected to an electrostatic effect.

In order to solve the above problem, there has been made such that, in a pulse measuring apparatus comprising a pulse sensor unit in which a pulse sensor has been accommodated in a sensor housing, a pulse measuring instrument at least possessing a display section displaying a pulse number, and an arm wearing band which has been fixed to the pulse measuring instrument and to which the pulse sensor unit has been attached so as to be capable of adjusting its position in a longitudinal direction, a radio transmission section has been possessed in the pulse sensor unit and a radio reception section in the measuring instrument, respectively, and a data is radio-transmitted from the pulse sensor unit to the measuring instrument. The data to be radio-transmitted is a pulse detection signal of the pulse sensor of the pulse sensor unit, or the pulse number having been obtained by calculation-processing the pulse detection signal by a signal processing section of the pulse sensor unit.

Embodiments of the present invention will now be described by way of further example only and with reference to the accompanying drawings, in which:
Fig. 1 is a plan view of a main portion of a wristwatch type pulse measuring apparatus of an embodiment 1, where a left side half of the drawing is shown under a state in which a cover glass of a timepiece head has been removed;
Fig. 2 is a sectional view of the timepiece head constituting the wristwatch type pulse measuring apparatus of the embodiment 1 along a band longitudinal direction;
Fig. 3 is a sectional view of the wristwatch type pulse measuring apparatus of the embodiment 1 under a state having been worn to an arm 8 along the band longitudinal direction;
Fig. 4 is an enlarged sectional view of a pulse sensor unit constituting the wristwatch type pulse measuring apparatus of the embodiment 1 along the band longitudinal direction;
Fig. 5 is a plan view of a main portion of a wristwatch type pulse measuring apparatus of an embodiment 2, where a left side half of the drawing is shown under the state in which the cover glass of the timepiece head has been removed;
Fig. 6 is a sectional view of the timepiece head constituting the wristwatch type pulse measuring apparatus of the embodiment 2 along the band longitudinal direction;
Fig. 7 is a sectional view of the wristwatch type pulse measuring apparatus of the embodiment 2 under the state having been worn to the arm 8 along the band longitudinal direction;
Fig. 8 is an enlarged sectional view of the pulse sensor unit constituting the wristwatch type pulse measuring apparatus of the embodiment 2 along the band longitudinal direction;
Fig. 9 is an enlarged sectional view of the pulse sensor unit constituting the wristwatch type pulse measuring apparatus of the embodiment 2 under a state having been attached to a band, which is a sectional view along a direction perpendicular to the band longitudinal direction;
Fig. 10 is a constitution diagram of the wristwatch type pulse measuring apparatus of the embodiment 1, in which a timepiece head as a pulse measuring instrument and the pulse sensor unit have been shown respectively by block circuits;
Fig. 11 is a constitution diagram of the wristwatch type pulse measuring apparatus of the embodiment 2, in which the timepiece head as the pulse measuring instrument and the pulse sensor unit have been shown respectively by block circuits; and
Fig. 12 is a constitution diagram of a conventional wristwatch type pulse measuring apparatus, in which the timepiece head as the pulse measuring instrument and the pulse sensor unit have been shown respectively by block circuits.

In an embodiment of a wristwatch type pulse measuring apparatus concerned with the present invention, in a wristwatch with a pulse number measuring function, which has a pulse sensor unit in which a pulse sensor has been accommodated in a sensor housing, and a timepiece case which has built in a calculation processing section calculating a pulse number by processing a signal of the pulse sensor and which has been attached to the band, a radio transmission section is possessed by the pulse sensor unit, a radio reception section is possessed by the timepiece case, and the signal of the pulse sensor of the pulse sensor unit is transmitted through the radio transmission section and the radio reception section, thereby inputting it to the calculation processing section.

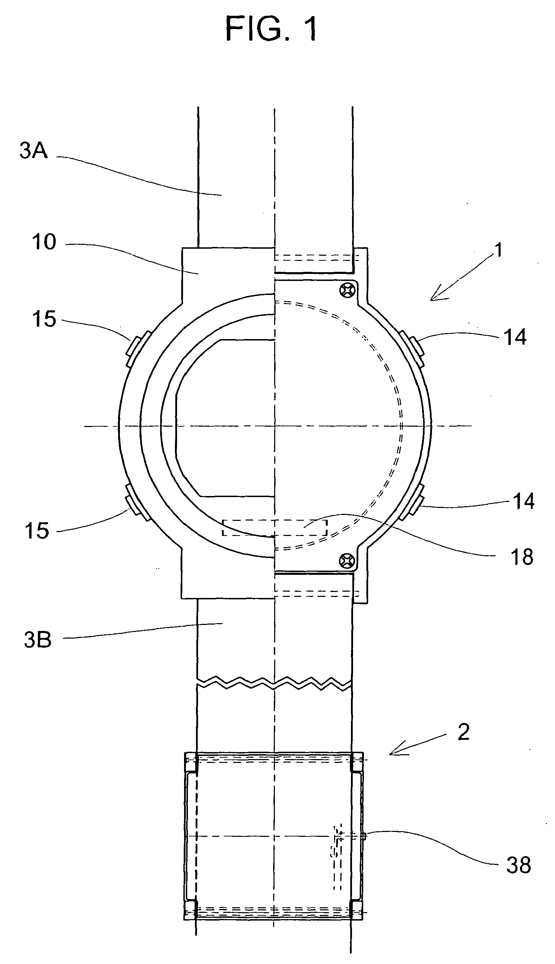

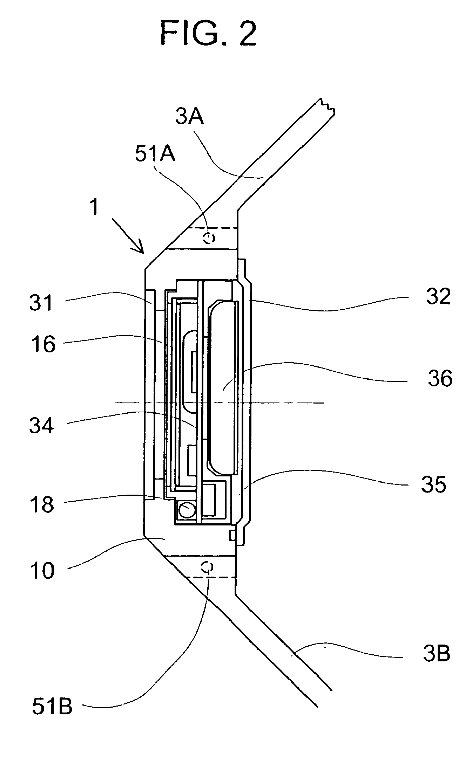

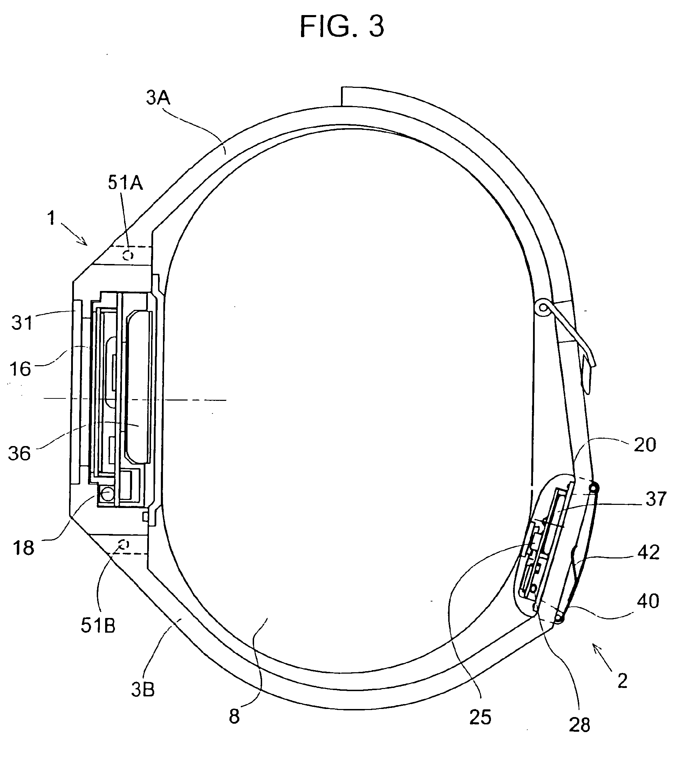

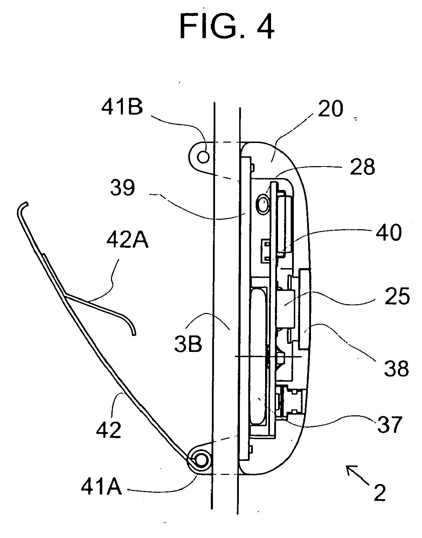

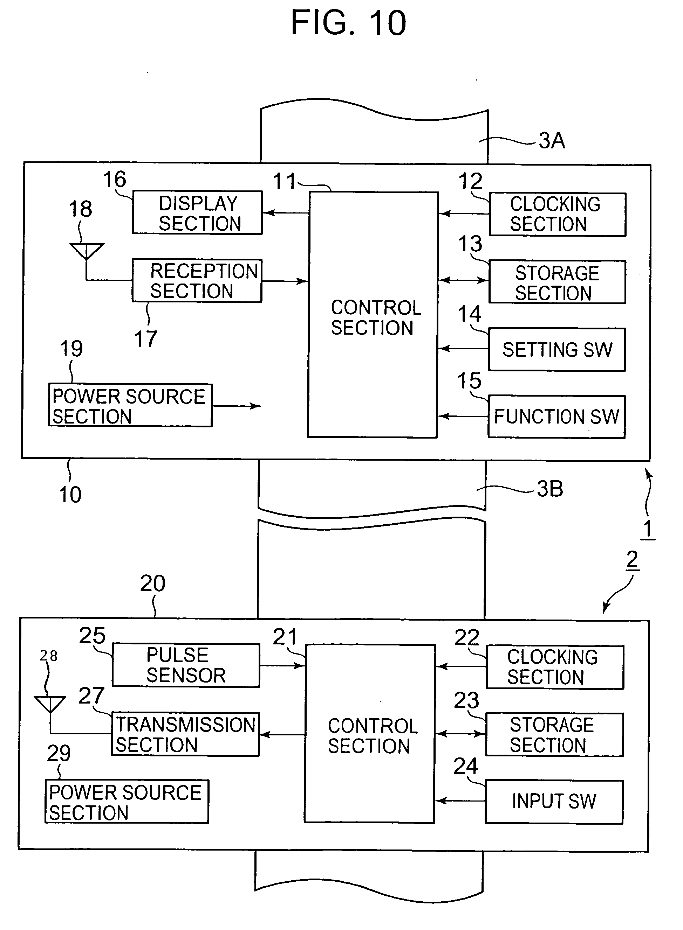

As shown in a plan view, of Fig. 1, of a main portion of a wristwatch type pulse measuring apparatus, a sectional view, of Fig. 2, of a timepiece head having been cut along a band longitudinal direction, a sectional view, of Fig. 3, of the wristwatch type pulse measuring apparatus having been shown while being cut along the band longitudinal direction under a state having been worn to a wrist 8, an enlarged sectional view, of Fig. 4, of a pulse sensor unit having been shown while being cut along the band longitudinal direction, and a block circuit diagram, of Fig. 10, of the wristwatch type pulse measuring apparatus, an embodiment 1 of the present invention is the wristwatch type pulse measuring apparatus having been constituted by the pulse sensor unit 2 in which the pulse sensor 25 has been accommodated in the sensor housing 20, the timepiece head 1 at least possessing the display section 16 displaying the pulse number, and a band comprising one pair of band pieces 3A and 3B, and the pulse sensor unit 2 is one having built in a power source.

That is, the wristwatch type pulse measuring apparatus of the embodiment 1 is one characterized in that, in the wristwatch type pulse measuring apparatus having been constituted by the pulse sensor unit 2 in which the pulse sensor 25 has been accommodated in the sensor housing 20, the timepiece head 1 at least possessing the display section 16 displaying the pulse number, and the band comprising one pair of band pieces 3A and 3B, a radio transmission section 27 is possessed in the pulse sensor unit 2 and a radio reception section 17 in the timepiece head 1, respectively, and the pulse sensor unit 2 builds in the power source. The pulse sensor 25 is a sensor including a light emitting element and a light receiving element. Differing from an embodiment 2 explained later, the sensor housing 20 is disposed in a back side of the band piece 3B.

As to the band comprising one pair of band pieces 3A and 3B, one end of the band piece 3A is attached to one band attaching part of a timepiece case 10 by a spring rod 51A, and one end of the band piece 3B is attached to one band attaching part of the timepiece case 10 by a spring rod 51B.

If shown by a block circuit, like Fig. 10, the wristwatch type pulse measuring apparatus of the embodiment 1 is one having been constituted by the timepiece head 1 functioning as the pulse measuring instrument, the pulse sensor unit 2 having built in the power source, and one pair of band pieces 3A and 3B. The timepiece head 1 is one in which the control section 11, the clocking section 12, the storage section 13, the setting switch 14 performing a time setting, the function switch 15 performing a mode switching and the like, the display section 16, a radio reception section 17, a reception antenna 18 and a power source section 19 have been accommodated in the timepiece case 10. Further, as shown in Fig. 10, the pulse sensor unit 2 is one in which a control section 21, a clocking section 22, a storage section 23, an input switch 24, the pulse sensor 25 including the light emitting element and the light receiving element, the radio transmission section 27, a transmission antenna 28 and a power source section 29 have been accommodated in the sensor housing 20.

As shown in Fig. 1 and Fig. 2, in the timepiece case 10 of the timepiece head 1, there are accommodated a circuit block 34 having been attached to a panel frame, the liquid crystal display panel 16 and the reception antenna 18, and a battery 36 having been held by a battery frame 35 is also accommodated. And, the timepiece case 10 is sealed in its front side opening by a cover glass 31, and sealed in its back side opening by a case back 32.

As shown in Fig. 3 and Fig. 4, in the sensor housing 20 of the pulse sensor unit 2, there are accommodated a circuit block 40 and the transmission antenna 28, which have been attached to a panel frame, the pulse sensor 25 including the light emitting element and the light receiving element, and a battery 37 having been held by a battery frame. In a central part of the sensor housing 20, there is formed an opening part having been sealed by a transparent head cover 38, and the pulse sensor 25 is disposed in a position, in the sensor housing 20, having corresponded to the above opening part.

As shown in Fig. 1 and Fig. 3, in the wristwatch type pulse measuring apparatus of the embodiment 1, the pulse sensor unit 2 contacts in the head cover 38 of its pulse sensor 25 with an artery position of the wrist 8 of the user, i.e., a surface suitable for the pulse detection, and is attached while being made adjustable in the longitudinal direction.

As shown in Fig. 3 and Fig. 4, the sensor housing 20 of the pulse sensor unit 2 is formed in its case back 39 side with a band insertion part having possessed a 1st protrusion 41A, a 2nd protrusion 41B and a fastener 42. The fastener 42 is a spring member whose one end has been rotatably attached to the 1st protrusion 41A, and is one having possessed an auxiliary spring member 42A in its band piece side. The sensor housing 20 of the pulse sensor unit 2 is inserted into the band insertion part at a predetermined position of the band piece 3B, and the other end of the fastener 42 is locked to the 2nd protrusion 41B. Thereupon, the pulse sensor unit 2 is properly fixed to the back side of the band piece 3B and to a predetermined pulse detection position while a spring action of the auxiliary spring member 42A acts as well.

If the user who has worn the wristwatch type pulse measuring apparatus of the embodiment 1 on the arm pushes the input switch 24 of the pulse sensor unit 2, it enters to a pulse measuring mode. That is, the pulse sensor unit 25 outputs a pulse detection signal to thereby input it to the control section 21. Thereupon, the control section 21 calculation-processes the pulse detection signal in compliance with a program stored in the storage section 23 to thereby output the number of pulses. Subsequently, the control section 21 causes the radio transmission section 27 to operate, thereby radio-transmitting the pulse number data from the antenna 28. In the timepiece head 1, the radio reception section 17 receives the pulse number data through the antenna 18, and inputs it to the control section 11. Thereupon, the control section 11 causes the display section 16 to operate, thereby displaying the pulse number. If the user pushes again the input switch 24 of the pulse sensor unit 2, the pulse measuring mode finishes.

As mentioned above, in the wristwatch type pulse measuring apparatus of the embodiment 1, there has been constituted such that the pulse sensor unit 2 calculation-processes the pulse detection signal of the pulse sensor 25 to the pulse number by its control section 21 to thereby transmit the pulse number from the radio transmission section 27, and the timepiece head 1 that is the pulse measuring instrument receives the pulse number by the radio reception section 17 to thereby display it by the display section 16.

However, in the wristwatch type pulse measuring apparatus of the embodiment 1, it is also possible to constitute such that the pulse sensor unit 2 transmits the pulse detection signal of the pulse sensor 25 from the radio transmission section 27, and the timepiece head 1 that is the pulse measuring instrument receives the pulse detection signal by the radio reception section 17, thereby calculation-processing it to the pulse number and displaying the pulse number by the display section 16.

By the way, by constituting the radio reception section 17 of the timepiece head 1 as a radio transmission/reception section, and constituting the radio transmission section 27 of the pulse sensor unit 2 as a radio transmission section, there can be also realized a switching of the pulse measuring mode having been made so as to control a starting of the pulse sensor unit 2 from the timepiece head 1. In this case, a switching order of the pulse measuring mode is inputted by an operation of the function switch 15 of the timepiece head, and a switching signal data of the pulse measuring mode is transmitted from the control section 11 through its transmission/reception section. And, it follows that a transmission/reception section of the pulse sensor unit 2 receives the switching signal data of the pulse measuring mode, thereby causing each constitutional element, such as the control section 21 and the pulse sensor 25, to operate.

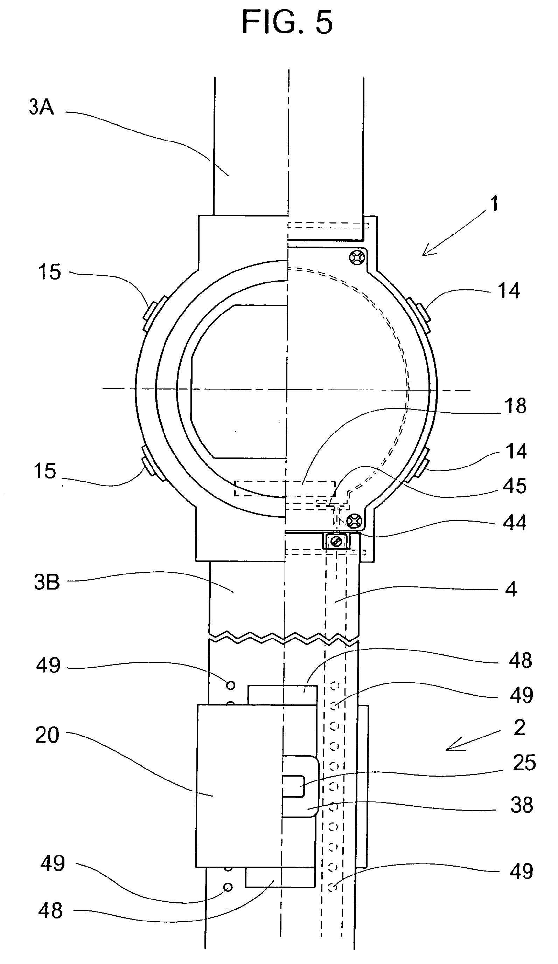

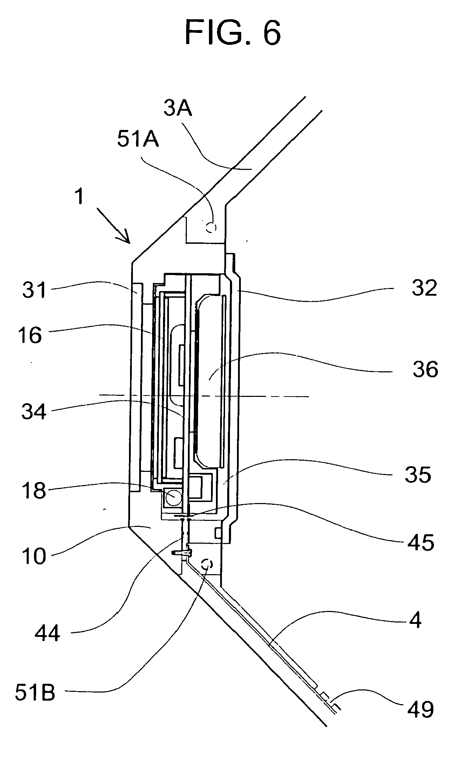

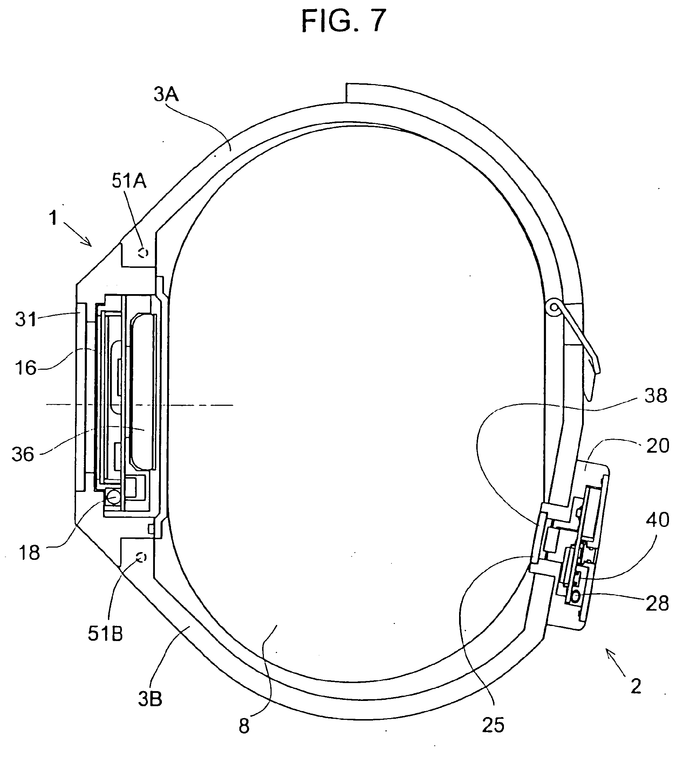

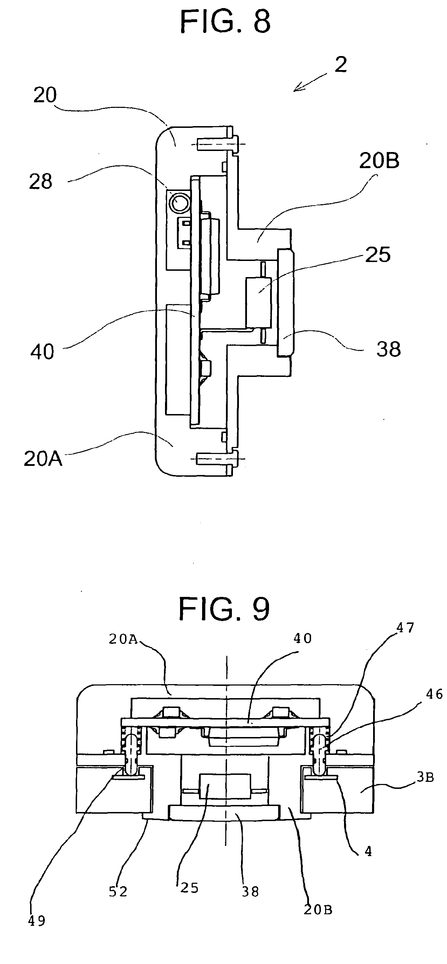

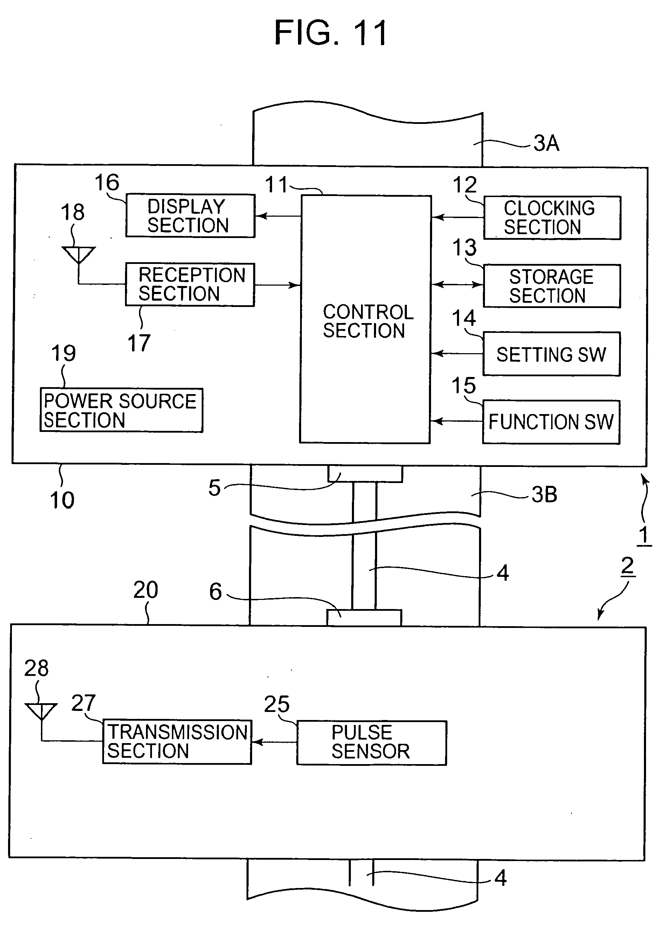

As shown in a plan view, of Fig. 5, of a main portion of a wristwatch type pulse measuring apparatus, a sectional view, of Fig. 6, of a timepiece head having been cut along a band longitudinal direction, a sectional view, of Fig. 7, of the wristwatch type pulse measuring apparatus having been shown while being cut along the band longitudinal direction under a state having been worn to the wrist 8, enlarged sectional views, of Fig. 8 and Fig. 9, of a pulse sensor unit having been shown while being cut along the band longitudinal direction, and a block circuit diagram, of Fig. 11, of the wristwatch type pulse measuring apparatus, an embodiment 2 of the present invention is the wristwatch type pulse measuring apparatus having been constituted by the pulse sensor unit 2 in which the pulse sensor 25 has been accommodated in the sensor housing 20, the timepiece head 1 at least possessing the display section 16 displaying the pulse number, and the band comprising one pair of band pieces 3A and 3B, and the pulse sensor unit 2 is one to which an electricity is supplied from the timepiece head 1.

That is, the wristwatch type pulse measuring apparatus of the embodiment 2 is one characterized in that, in the wristwatch type pulse measuring apparatus having been constituted by the pulse sensor unit 2 in which the pulse sensor 25 has been accommodated in the sensor housing 20, a pulse measuring instrument, i.e., the timepiece head 1, at least possessing the liquid crystal display panel 16 displaying the pulse number, and the band comprising one pair of band pieces 3A and 3B, the radio transmission section 27 has been possessed in the pulse sensor unit 2 and the radio reception section 17 in the timepiece head 1, respectively, and a wiring 4 for power source, which supplies the electricity to the pulse sensor unit 2 from the power source section of the timepiece head 1, has been possessed in the band 3B. The pulse sensor 25 is the sensor including the light emitting element and the light receiving element. There is constituted such that, differing from the embodiment 1, the sensor housing 20 is disposed in a front side of the band 3B, and only a sensor face of the pulse sensor unit 2 is exposed to the back side of the band 3B.

As to the band comprising one pair of band pieces 3A and 3B, one end of the band piece 3A is attached to one band attaching part of the timepiece case 10 by the spring rod 51A, and one end of the band piece 3B is attached to one band attaching part of the timepiece case 10 by the spring rod 51B.

As shown by a block circuit, like Fig. 11, the wristwatch type pulse measuring apparatus of the embodiment 2 is one having been constituted by the timepiece head 1 functioning as the pulse measuring instrument, the pulse sensor unit 2, one pair of band pieces 3A and 3B, and the wiring 4 for power source. The wiring 4 for power source is connected to the timepiece head 1 by a fixation connection part 5 and to the pulse sensor unit 2 by a position variable connection part 6, respectively. The timepiece head 1 is one in which the control section 11, the clocking section 12, the storage section 13, the setting switch 14 performing the time setting, the function switch 15 performing the mode switching and the like, the display section 16, the radio reception section 17, the reception antenna 18, and the power source section 19 have been accommodated in the timepiece case 10. Further, the pulse sensor unit 2 is one in which the pulse sensor 25 including the light emitting element and the light receiving element, the radio transmission section 27, and the transmission antenna 28 have been accommodated in the sensor housing 20.

As shown in Fig. 5 and Fig. 6, in the timepiece case 10 of the timepiece head 1, there are accommodated the circuit block 34 having been attached to the panel frame, a liquid crystal display panel 16 and the reception antenna 18, and the battery 36 having been held by the battery frame 35 is also accommodated. And, the timepiece case 10 is sealed in its front side opening by the cover glass 31, and sealed in its back side opening by the case back 32.

As shown in Fig. 7 to Fig. 9, the sensor housing 20 of the pulse sensor unit 2 is constituted by a front case 20A becoming a main body part, and a back case 20B having in its center a cylindrical protrusion part. In the front case 20A, there are accommodated the circuit block 40 having been attached to the panel frame and the transmission antenna 28 and, in the back case 20B, there is accommodated the pulse sensor 25 including the light emitting element and the light receiving element. An opening part of the protrusion part of the back case 20B is sealed by the transparent head cover 38.

As to the sensor housing 20, the front case 20A of the sensor housing 20 is disposed in a front side of the band piece 3B , the protrusion part of the back case 20B of the sensor housing 20 is penetrated through a slit 48 of the band piece 3B, and the transparent head cover 38 is attached to the band piece 3B so as to slightly protrude from a back face of the band piece 3B. The slit 48 is a through-hole for exposing a front face, i.e. , sensor face, of the protrusion part of the back case 20B, that is an accommodation part of the pulse sensor 25, to a back side of the band.

The slit 48 extends to the band piece 3B in the longitudinal direction and is formed as a rectangular through-hole. Accordingly, the pulse sensor unit 2 becomes such that an attaching position to the band piece 3B is adjustable in a range of a length of the slit 48 in the longitudinal direction.

In both sides of the slit 48 in a front face of the band piece 3B, plural small concave parts 49 are formed at an equal interval in the longitudinal direction. A diameter of the concave part 49 is slightly larger than a diameter of an electrically conductive connection pin 46, and the intervals between the adjoining concave parts 49 are respectively selected to the intervals suitable for an attaching position adjustment to the band piece 3B of the pulse sensor unit 2.

The wiring 4 for power source, which supplies the drive electricity to the pulse sensor unit 2 from the electric source section 19 of the timepiece head 1, is attached to the band piece 3B while extending from end to end of the slit 48 from the fixation connection part of the timepiece head 1 that is the pulse measuring instrument. The wiring 4 for power source is completely embedded in the band piece 3B from the fixation connection part till one end of the slit 48, and embedded in the band piece 3B so as to be partially exposed from one end till the other end of the slit 48. A connection between the wiring 4 for power source and a printed circuit of the circuit block 34 of the timepiece head 1 is performed by a connection pin 44 and a conduction spring 45. A connection part by the connection pin 44 and the conduction spring 45 constitutes the fixation connection part 5 of Fig. 11. Incidentally, the electricity is supplied to the printed circuit of the circuit block 34 from the power source 19.

An attachment of the wiring 4 for power source to the band piece 3B, which accompanies the partial exposure from one end to the other end of the slit 48, is performed by exposing the wiring 4 for power source to bottom faces of the concave parts 49 for position adjustment, which are formed at the equal interval in the longitudinal direction in both sides of the slit 48.

A connection between a printed wiring of the circuit block 40 of the pulse sensor unit 2 and the wiring 4 for power source is performed by using one pair of electrically conductive connection pins 46 and one pair of electrically conductive pressurized springs 47, which have been disposed in the sensor housing 20. That is, as shown in Fig. 9, the electrically conductive connection pins 46 having been inserted into one pair of guide holes having been provided in the sensor housing 20 are connected in their rear end parts to the circuit block 40 through the electrically conductive pressurized springs 47, and are contacted in their tip parts with the wiring 4 for power source, which is exposed to a lower face of the concave part 49 while being pressurized from the electrically conductive pressurized spring 47. The one pair of guide holes are formed in positions adjoining both sides of the cylindrical protrusion part in the center of the back case 20B of the sensor housing 20, i.e., positions corresponding to the plural small concave parts 49 formed in both sides of the slit 48 in the front face of the band piece 3B.

In short, the position variable connection part (the position variable connection part 6 of Fig. 11) connecting the wiring 4 for power source to the pulse sensor unit 2 is constituted by the exposed part of the wiring 4 for power source, which has been formed in the bottom face of the concave part 49 for position adjustment, which is formed at the equal interval in the longitudinal direction in both side of the slit 48, and the one pair of electrically conductive connection pins 46 and the one pair of electrically conductive pressurized springs 47, which have been disposed in the sensor housing 20.

In a tip part of the protrusion part of the back case 20B that is a pulse sensor accommodation part of the sensor housing 20, there is formed an eaves part 52 for holding, which extends along a side face of the slit 48 in the longitudinal direction. Accordingly, the band piece 3B is nipped between the back face of the sensor housing 20 and the eaves part 52 for holding. By this, the sensor unit 2 is held by the band piece 3B, and fixed to that position. And, the connection pin 46 electrically connecting the wiring 4 for power source and the circuit block 40 of the pulse sensor unit 2 is accommodated at its tip part by the concave part 49, and contacts with its lower face while being pressurized from the exposed electrically conductive pressurized spring 47. In this manner, while holding the connection between the wiring 4 for power source and the circuit block 40 of the pulse sensor unit 2, the attaching position of the band piece 3B of the sensor unit 2 in the longitudinal direction becomes adjustable for every disposition interval of the concave part 49 in the range of the length of the slit 48 in the longitudinal direction.

In the wristwatch type pulse measuring apparatus of the embodiment 2, the pulse sensor unit 2 is made such that the transparent head cover 38 of its pulse sensor 25 contacts with the artery position of the user's wrist, i.e., the surface suitable for the pulse detection, and attached while being made adjustable in the longitudinal direction. In comparison with the embodiment 1, in the wristwatch type pulse measuring apparatus of the embodiment 2, a contact area between the band and the wrist 8 becomes wide, so that there is an advantage that a natural wear feeling is obtained.

In one variation, if the user who has worn the wristwatch type pulse measuring apparatus of the embodiment 2 to the arm pushes an input switch (not shown in the drawing) of the pulse sensor unit 2, it enters into the pulse measuring mode. That is, the pulse sensor 25 outputs the pulse detection signal, thereby inputting it to the control section 21. Thereupon, the control section 21 causes the radio transmission section 27 to operate, thereby radio-transmitting the pulse detection signal data from the antenna 28. In the timepiece head 1, the radio reception section 17 receives the pulse detection signal data through the antenna 18, thereby inputting it to the control section 11. Thereupon, the control section 11 calculation-processes the pulse detection signal data in compliance with the program stored in the storage section 13, thereby outputting the pulse number. Subsequently, the control section 11 causes the display section 16 to operate, thereby displaying the pulse number. If the user pushes again the input switch (not shown in the drawing) of the pulse sensor unit 2, the pulse measuring mode finishes.

As mentioned above, in the wristwatch type pulse measuring apparatus of the embodiment 2, there has been constituted such that the pulse sensor unit 2 transmits the pulse detection signal of the pulse sensor 25 from the radio transmission section 27, and the timepiece head 1 that is the pulse measuring instrument receives the pulse detection signal by the radio reception section 17, thereby calculation-processing it to the pulse number by the control section and displaying the pulse number by the display section 16.

However, in the wristwatch type pulse measuring apparatus of the embodiment 2, it is also possible to make such that the pulse sensor unit 2 calculation-processes the pulse detection signal of the pulse sensor 25 to the pulse number, thereby transmitting the pulse number from the radio transmission section 27, and to constitute such that the timepiece head 1 that is the pulse measuring instrument receives the pulse number by the radio reception section 17, thereby displaying it by the display section 16.

By the way, in the wristwatch type pulse measuring apparatus of the embodiment 2, by operating the function switch 15 of the timepiece head, it is also possible so as to perform the switching of the pulse measuring mode. For example, by inserting an electronic switch into the wiring 4 for power source, there can be realized by constituting such that the control section 11 controls an ON/OFF of the electronic switch by an input of the function switch 15.

By the present invention, there has been made possible such that, in a pulse measuring apparatus comprising a pulse sensor unit in which a pulse sensor has been accommodated in a sensor housing, a pulse measuring instrument at least possessing a display section displaying a pulse number, and an arm wearing band which has been fixed to the pulse measuring instrument and to which the pulse sensor unit has been attached so as to be adjustable in a longitudinal direction, a signal of the pulse sensor unit is certainly transmitted to the pulse measuring instrument without being subjected to the electrostatic effect and without the signal transmission means being subjected to the physical adverse effect. Accordingly, by the present invention, there has been provided a wristwatch type pulse measuring apparatus which is not subjected to the effects with respect to external environments such as static electricity and humidity, whose reliability is high, and whose portability is good as well.

It should be noted that the term "wristwatch" in the foregoing description neither precludes nor requires a time-keeping or displaying function.

The aforegoing description has been given by way of example only and it will be appreciated by a person skilled in the art that modifications can be made without departing from the scope of the present invention.

## Claims

1. A pulse measuring apparatus comprising:
a pulse sensor unit in which a pulse sensor has been accommodated in a sensor housing;
a pulse measuring instrument at least possessing a display section displaying a pulse number; and
an arm wearing band which has been fixed to the pulse measuring instrument and to which the pulse sensor unit has been attached so as to be capable of adjusting its position in a longitudinal direction **characterized in that** a radio transmission section has been possessed in the pulse sensor unit and a radio reception section in the pulse measuring instrument, respectively.

2. A pulse measuring apparatus according to claim 1; wherein the pulse sensor unit is one which transmits a pulse detection signal of the pulse sensor, and the pulse measuring instrument is one which receives the pulse detection signal by the radio reception section and calculation-processes it to a pulse number by its signal processing section, thereby displaying the pulse number by the display section.

3. A pulse measuring apparatus according to claim 1;
wherein the pulse sensor unit is one which calculation-processes a pulse detection signal of the pulse sensor to a pulse number by its signal processing section to thereby transmit the pulse number from the radio transmission section, and the pulse measuring instrument is one which receives the pulse number by the radio reception section, thereby displaying it by the display section.

4. A pulse measuring apparatus according to any one of the preceding claims, wherein a power source of the pulse sensor unit is a battery having been accommodated in the sensor housing.

5. A pulse measuring apparatus according to any one of claims 1 to 3,wherein a drive voltage is supplied to the pulse sensor by a wiring for power source, which has been provided in the band, from a battery accommodated in a measuring instrument case.

6. A pulse measuring apparatus according to claim 5, wherein the wiring for power source comprises a pair of embedded lines having exposure parts which have been partially exposed in an attaching position of the pulse sensor unit, and movable power source terminals of the pulse sensor unit are disposed so as to electrically contact with the exposure parts of the wiring for power source.

7. A pulse measuring apparatus according to claim 6, wherein the attaching position of the pulse sensor unit is a slit having been formed in the band, and the wiring for power source has the exposure parts in bottom faces of concave parts for position adjustment, which are formed in both sides of the slit in a longitudinal direction.

8. A pulse measuring apparatus according to claim 6 or claim 7
wherein the movable power source terminals of the pulse sensor unit comprise a pair of electrically conductive connection pins and a pair of electrically conductive pressurized springs, which have been disposed in the sensor housing.

9. A pulse measuring apparatus according to any one of the preceding claims, wherein there has been provided an input means which instructs a starting and a stopping of a pulse detection to the pulse sensor unit.
